# EUROPEAN PATENT APPLICATION

(11) **EP 4 272 586 A1**
(43) Date of publication of application: **08.11.2023**
(21) Application number: 21914675.0
(22) Date of filing: 30.12.2021
(51) Int. Cl.: A24F 40/40, A24F 40/42, A24F 40/48, A24F 47/00, A61M 11/04

(54) **ATOMIZER AND ELECTRONIC ATOMIZATION DEVICE**

(30) Priority: 30.12.2020 CN 202011611873
(71) Applicant: Jiangmen Moore Technology., Ltd, Jiangmen, Guangdong 529080 (CN)
(72) Inventor: CHEN, Zhouwei, Jiangmen, Guangdong 529080 (CN); ZHANG, Chao, Jiangmen, Guangdong 529080 (CN); LI, Guanghui, Jiangmen, Guangdong 529080 (CN)
(74) Representative: De Arpe Tejero, Manuel
(86) International application number: PCT/CN2021/143303
(87) International publication number: WO 2022/143946

(57) **Abstract**

An atomizer (10) and an electronic atomization device (20). The atomizer (10) comprises an atomization sleeve (110), a mounting base (120), and an atomization core (130); the atomization sleeve (110) is provided with an e-liquid storage cavity (111) and a mounting cavity (112) therein; the mounting base (120) is provided in the mounting cavity (112); an atomization cavity (121) and an air inlet channel (122) and an air outlet channel (123) that are communicated with the atomization cavity (121) are formed on the mounting base; the atomization core (130) is mounted in the atomization cavity (121); a thin groove (1221) is provided on the inner wall of the air inlet channel (122); the thin groove (1221) is used for storing the e-liquid entering the air inlet channel (122). The thin groove (1221) is provided on the inner wall of the air inlet channel (122), such that the e-liquid entering the air inlet channel (122) can be stored by means of the thin groove (1221), and the risk of liquid leakage from the atomizer (10) is reduced.

## Description

### TECHNICAL FIELD

The present disclosure relates to the technical field of electronic atomization devices, and in particular, to an atomizer and an electronic atomization device.

### BACKGROUND

In the related art, an electronic atomization device generally includes an atomizer and a power supply assembly. A liquid storage cavity and an atomization cavity are generally provided in the atomizer. The liquid storage cavity is configured to store an atomizable medium, and an atomization assembly may be arranged in the atomization cavity for heating and atomizing the atomizable medium to form aerosol that can be inhaled by an inhaler. The power supply assembly is configured to supply power to the atomizer.

An air inlet channel and an air outlet channel that are in communication with the atomization cavity further need to be arranged in the atomizer, and the air outlet channel may be in communication with a suction nozzle for the inhaler to inhale aerosol in the atomization cavity. The air inlet channel may be in communication with the atomization cavity and the external air. In this way, when the inhaler inhales, the external air enters the atomization cavity through the air inlet channel, and aerosol generated by atomization of the atomizer is driven by an airflow and delivered to the inhaler.

The existing electronic atomization device is prone to have a problem of leakage of condensate of an atomization medium from the air inlet channel after being used for a period of time.

### SUMMARY

The present disclosure provides an electronic atomization device and an atomizer thereof, to resolve the foregoing technical problem.

To resolve the foregoing technical problem, a technical solution adopted in the present disclosure is to provide an atomizer, including: an atomization sleeve, wherein a liquid storage cavity and a mounting cavity are provided on the atomization sleeve; a mounting base, wherein the mounting base is arranged in the mounting cavity, an atomization cavity, an air inlet channel in communication with the atomization cavity, and an air outlet channel in communication with the atomization cavity are provided on the mounting base; and an atomization core, wherein the atomization core is mounted in the atomization cavity; wherein at least one fine slot is provided on the inner wall of the air inlet channel, and the at least one fine slot is configured to buffer liquid entering the air inlet channel.

In some embodiments, the mounting base includes a base and a sealing member, the sealing member is arranged on the side of the base close to the liquid storage cavity, and is engaged with the base, and the sealing member and the base are enclosed to form the atomization cavity.

In some embodiments, an air guiding groove is provided on the outer surface of the side wall of the base, the air inlet channel is formed by the air guiding groove and the side wall of the mounting cavity cooperatively, the end of the air inlet channel close to the liquid storage cavity is in communication with the atomization cavity, and the end of the air inlet channel away from the liquid storage cavity is in communication with external air; and the at least one fine slot is provided in the air guiding groove.

In some embodiments, the air guiding groove is extended along the side of the mounting base close to the liquid storage cavity to the side away from the liquid storage cavity, and the at least one fine slot is provided on the bottom wall of the air guiding groove and the width of the at least one fine slot ranges from 0.2 mm to 1.2 mm.

In some embodiments, the air guiding groove is extended along the side of the mounting base close to the liquid storage cavity to the side away from the liquid storage cavity, and the at least one fine slot is provided on the bottom wall of the air guiding groove and the width of the at least one fine slot ranges from 0.3 mm to 0.7 mm.

In some embodiments, a first intercepting groove is provided on the side of the air guiding groove close to the liquid storage cavity, and the at least one fine slot is in communication with the atomization cavity through the first intercepting groove.

In some embodiments, a second intercepting groove is provided on the side of the air guiding groove away from the liquid storage cavity, and the at least one fine slot is in communication with the external air through the second intercepting groove.

In some embodiments, at least one third intercepting groove is provided in the middle portion of the air guiding groove, and the third intercepting groove cuts off the at least one fine slot.

In some embodiments, the first intercepting groove is a first notch on the edge of the side wall of the base close to the liquid storage cavity, and the second intercepting groove is a second notch on the edge of the bottom wall of the mounting base.

In some embodiments, a baffle plate is arranged between the air inlet channel and the atomization cavity; the baffle plate is configured to close the air inlet channel in response to no air being inhaled from the atomization cavity through the air outlet channel, and disconnect the communication between the air inlet channel and the atomization cavity; and the baffle plate is configured to open the air inlet channel in response to air being inhaled from the atomization cavity through the air outlet channel, to communicate the air inlet channel with the atomization cavity.

In some embodiments, an engagement groove is provided on the side wall of the sealing member, and the engagement groove is sleeved on the side wall of the base; and the side wall on one side of the engagement groove is arranged between the air inlet channel and the side wall of the mounting cavity, and the side wall on the other side of the engagement groove is arranged between the atomization core and the side wall of the base.

In some embodiments, the baffle plate is fixed to the bottom wall of the engagement groove of the sealing member, and the baffle plate is integrally formed with the sealing member.

In some embodiments, the cross section of the atomization cavity has two opposite long sides and two opposite short sides, and the air inlet channel is located on either end of the long sides.

To resolve the foregoing technical problem, a technical solution adopted in the present disclosure is to provide an electronic atomization device, including: an atomizer, wherein the atomizer is configured to store liquid to be atomized and atomize the liquid to form an aerosol for inhalation by an inhaler, and the atomizer is the aforesaid atomizer; and a body assembly, wherein the body assembly is configured to supply power to the atomizer.

Beneficial effects of the present disclosure are as follows: the present disclosure provides an electronic atomization device and an atomizer thereof. By providing at least one fine slot in an air inlet channel, condensed atomized liquid in the air inlet channel may enter the at least one fine slot for buffering, thereby preventing the condensed atomized liquid from leaking out from an air inlet of the air inlet channel. Further, by providing the air inlet channel on either end of long sides of the cross section of an atomization cavity, when an atomization core is mounted in the atomization cavity, a path of passing an atomization surface of the atomization core performed by an airflow entering the atomization cavity from the air inlet channel may be lengthened, thereby increasing a heating time of the airflow. In this way, condensate of atomized liquid and splashed atomized liquid generated in an atomizer may be fully absorbed or heated and atomized by the atomization core, reducing occurrence of a phenomenon of inhalation leakage and condensate accumulation.

### BRIEF DESCRIPTION OF THE DRAWINGS

FIG. 1 is a schematic structural view of an embodiment of an atomizer according to the present disclosure.
FIG. 2 is a schematic, exploded structural view of the atomizer shown in FIG. 1.
FIG. 3 is a cross-sectional view of the atomizer shown in FIG. 1.
FIG. 4 is a schematic structural view of the base shown in FIG. 2.
FIG. 5 is a schematic structural view of the base shown in FIG. 4 from another perspective.
FIG. 6 is a schematic structural view of an embodiment of the sealing member shown in FIG. 2.
FIG. 7 is a cross-sectional view of the sealing member shown in FIG. 6.
FIG. 8 is a schematic structural view of an embodiment of an electronic atomization device according to the present disclosure.

### DETAILED DESCRIPTION

In order to make the objects, technical solutions and advantages of embodiments of the present disclosure clearer, technical solutions of the embodiments of the present disclosure will be clearly and comprehensively described in the following with reference to the accompanying figures in the embodiments of the present disclosure. It is apparent that the described embodiments are a part of the embodiments of the present disclosure, rather than all of the embodiments. All other embodiments obtained by a person skilled in the art based on the embodiments of the present disclosure without creative efforts shall fall within the protection scope of the present disclosure.

In the present disclosure, the terms "first", "second" and "third" are used merely for the purpose of description, and shall not be construed as indicating or implying relative importance or implying a quantity of indicated technical features. Therefore, features defining "first" "second" and "third" can explicitly or implicitly include at least one of the features. In description of the present disclosure, "more" means at least two, such as two and three unless it is specifically defined otherwise. All directional indications (for example, up, down, left, right, front, back) in the embodiments of the present disclosure are only used for explaining relative position relationships, movement situations, or the like between the various components in a specific posture (as shown in the accompanying drawings). If the specific posture changes, the directional indications change accordingly. In addition, the terms "include", "have", and any variant thereof are intended to cover a non-exclusive inclusion. For example, a process, method, system, product, or device that includes a series of steps or units is not limited to the listed steps or units; and instead, further optionally includes a step or unit that is not listed, or further optionally includes another step or unit that is intrinsic to the process, method, product, or device.

"Embodiment" mentioned in the specification means that particular features, structures, or characteristics described with reference to the embodiment may be included in at least one embodiment of the present disclosure. The term appearing at different positions of the specification may not refer to the same embodiment or an independent or alternative embodiment that is mutually exclusive with another embodiment. A person skilled in the art explicitly or implicitly understands that the embodiments described in the specification may be combined with other embodiments in the absence of conflict. Detailed descriptions are provided below respectively with reference to specific embodiments.

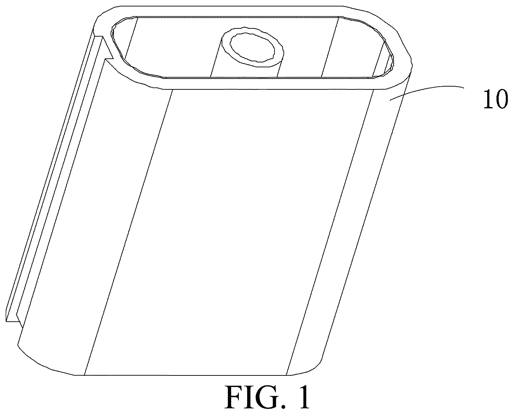

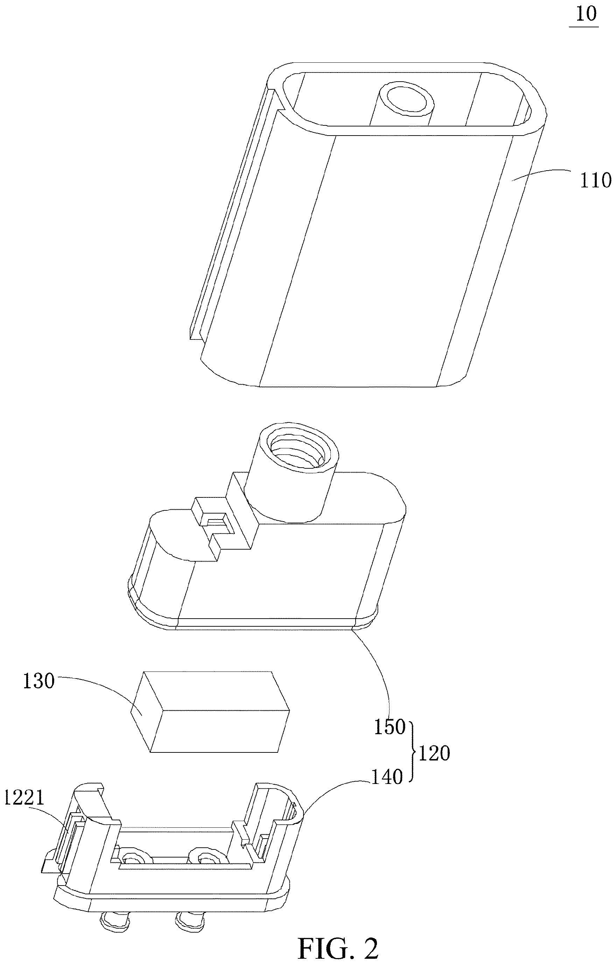

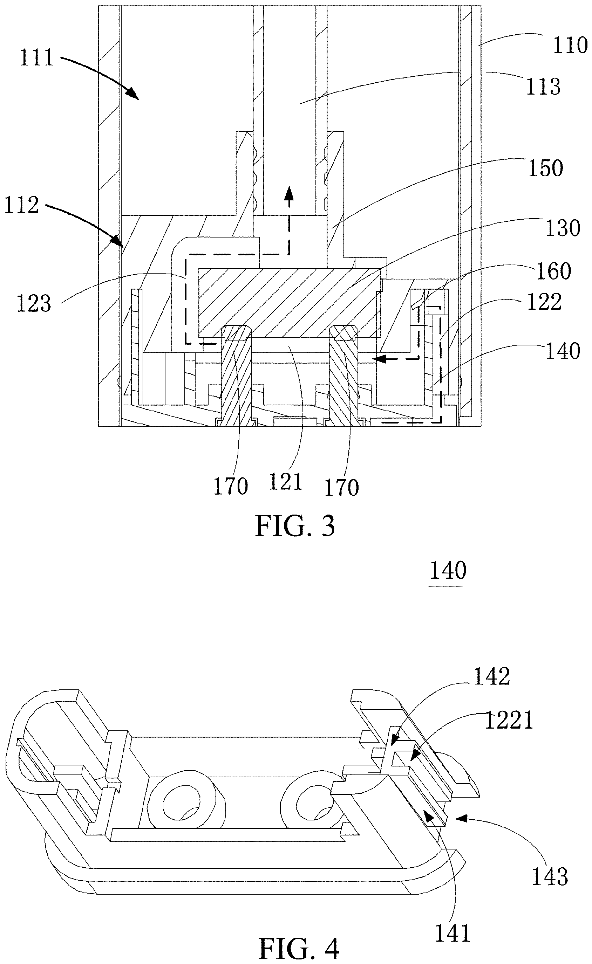

As shown in FIGS. 1 to 3, FIG. 1 is a schematic structural view of an embodiment of an atomizer according to the present disclosure, FIG. 2 is a schematic, exploded structural view of the atomizer shown in FIG. 1, and FIG. 3 is a cross-sectional view of the atomizer shown in FIG. 1.

An atomizer 10 includes an atomization sleeve 110, a mounting base 120, and an atomization core 130.

A liquid storage cavity 111 and a mounting cavity 112 are provided on the atomization sleeve 110; and the mounting base 120 is arranged in the mounting cavity 112, and an atomization cavity 121, an air inlet channel 122 in communication with the atomization cavity 121, and an air outlet channel 123 in communication with the atomization cavity 121 are provided on the mounting base 120. An atomization core 130 is mounted in the atomization cavity 121, and may be in communication with the liquid storage cavity 111, in this way, the atomization core 130 in the atomization cavity 121 may receive atomized liquid stored in the liquid storage cavity 111 and heat and atomize the atomized liquid, and the atomized aerosol of the atomized liquid may be formed in the atomization cavity 121.

The air inlet channel 122 may communicate the atomization cavity 121 with external air, and may be configured to supply air to the atomization cavity 121; and the air outlet channel 123 may be in communication with a preset suction nozzle. When an inhaler inhales air through the air outlet channel 123, air in the atomization cavity 121 may be sucked out so that air pressure in the atomization cavity 121 is lower than the atmospheric pressure. Through the action of a pressure difference, the external air may enter the atomization cavity 121 from the air inlet channel 122, and be sucked out through the air outlet channel 123, thereby forming an airflow, and the atomized aerosol of the atomized liquid may be sucked out with the movement of the airflow.

When the inhaler stops inhaling air, an air backflow may occur in the atomization cavity 121, so that the aerosol in the atomization cavity 121 enters the air inlet channel 122. The aerosol entering the air inlet channel 122 condenses to form atomized liquid, and may leak out from an air inlet of the air inlet channel 122.

In some embodiments, by providing at least one fine slot 1221 on the inner wall of an air inlet channel 122, condensed atomized liquid in the air inlet channel 122 may enter the at least one fine slot 1221 for buffering, thereby preventing the condensed atomized liquid from leaking out from an air inlet of the air inlet channel 122.

The atomized liquid may be absorbed under surface tension of the atomized liquid and stored in the at least one fine slot 1221, and the width of the at least one fine slot 1221 may be set to range from 0.2 mm to 1.2 mm, for example, may be set to 0.2 mm, 0.7 mm, or 1.2 mm. In some embodiments, the width of the at least one fine slot 1221 may be set to range from 0.3 mm to 0.7 mm, for example, may be set to 0.3 mm, 0.5 mm, or 0.7 mm.

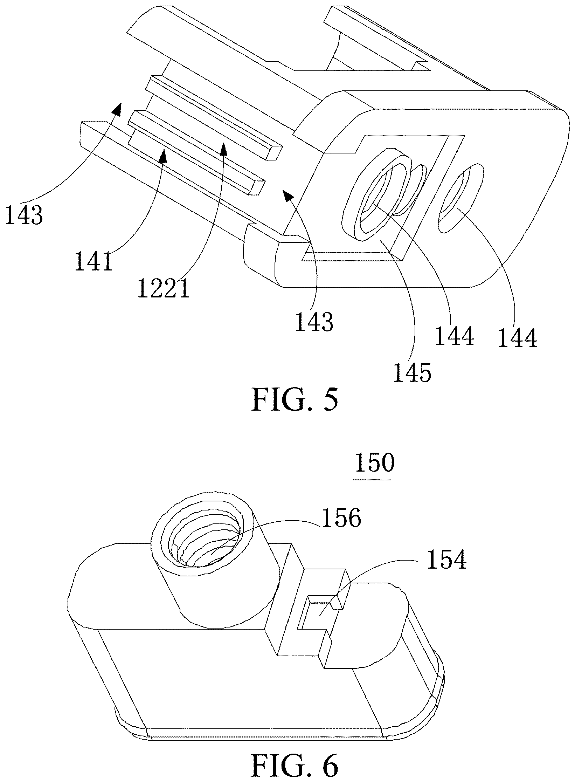

Further, as shown in FIGS. 2, 4, and 5, FIG. 4 is a schematic structural view of the base shown in FIG. 2, and FIG. 5 is a schematic structural view of the base shown in FIG. 4 from another perspective.

In some embodiments, the mounting base 120 may be at least partially inserted into an atomization sleeve 110 for sealing the atomization sleeve 110.

The mounting base 120 includes a base 140 and a sealing member 150. The sealing member 150 may be arranged on the side of the base 140 close to the liquid storage cavity 111, and is engaged with the base 140. The sealing member 150 and the base 140 are enclosed form the atomization cavity 121. An air guiding groove 141 is provided on the outer surface of the side wall of the base 140, and the air inlet channel 122 is formed by the air guiding groove 141 and the side wall of a mounting cavity 112 (or the side wall of the sealing member 150)cooperatively; and the end of the air inlet channel 122 close to the liquid storage cavity 111 is in communication with the atomization cavity 121, and the end of the air inlet channel 122 away from the liquid storage cavity 111 is in communication with the external air, and the at least one fine slot 1221 may be provided in the air guiding groove 141.

In some embodiments, at least two protruding walls spaced apart from each other are arranged in the air guiding groove 141, and the two adjacent protruding walls may be enclosed to form the at least one fine slot 1221. In some embodiments, the at least one fine slot 1221 may also be a groove provided on the inner wall of the air guiding groove 141.

An extension direction of the at least one fine slot 1221 may be arranged parallel to an extension direction of the air guiding groove 141. A quantity of at least one fine slots 1221 may also be one or at least two, and at least two at least one fine slots 1221 may be spaced apart from each other in the air guiding groove 141.

In some embodiments, the at least one fine slot 1221 may be extended from the outer surface of the side wall of the base 140 into the atomization cavity 121.

A first intercepting groove 142 is provided on the side of the air guiding groove 141 close to the liquid storage cavity 111, and the at least one fine slot 1221 is in communication with the atomization cavity 121 through the first intercepting groove 142.

In some embodiments, the first intercepting groove 142 may cut off the at least one fine slot 1121 and the inner wall of the atomization cavity 121; and the first intercepting groove 142 may be a first notch on the side wall of the base 140 close to the edge of the liquid storage cavity 141, and the first intercepting groove 142 may cut off the at least one fine slot 1221, thereby preventing a problem of liquid leakage caused by liquid in the atomization cavity 121 flowing out along the at least one fine slot 1221 due to capillary action.

A second intercepting groove 143 is provided on the side of the air guiding groove 141 away from the liquid storage cavity 111, and the at least one fine slot 1221 may be in communication with the external air through the second intercepting groove 143. In some embodiments, the second intercepting groove 143 is a second notch on the edge of the bottom wall of the mounting base 120, and the second intercepting groove 143 may cut off a connection between the at least one fine slot 1221 and the inner wall on which an air inlet of the external air is located.

In some embodiments, a third intercepting groove (which are not shown in the figures) may further be provided in the air guiding groove 141 on the side wall of the base 140, the third intercepting groove may further cut off the at least one fine slot 1221 on the side wall of the base 140. A quantity of third intercepting grooves is one or more.

In some embodiments, the cross section of the atomization cavity 121 may be rectangular or approximately rectangular, and has two opposite long sides and two opposite short sides. The air inlet channel 122 and the air outlet channel 123 may be respectively located on two opposite ends of the long sides. When the atomization core 130 is mounted in the atomization cavity 121, long sides of an atomization surface of the atomization core 130 may be arranged along the long sides of the cross section of the atomization cavity 121.

Therefore, by respectively providing the air inlet channel 122 and the air outlet channel 123 on two opposite ends of the long sides of the cross section of the atomization cavity 121, when an atomization core 130 is mounted in the atomization cavity 121, a path of passing an atomization surface of the atomization core 130 performed by an airflow entering the atomization cavity 121 from the air inlet channel 122 may be lengthened, thereby increasing a heating time of the airflow. In this way, condensate of atomized liquid and splashed atomized liquid generated in an atomizer 10 may be fully absorbed or heated and atomized by the atomization core 130, thereby reducing a phenomenon of inhalation leakage and condensate accumulation.

To further improve the sealing effect of the atomizer 10, and reduce the occurrence of a problem of the liquid leakage of the atomizer 10, a baffle plate may further be arranged in the first intercepting groove 142.

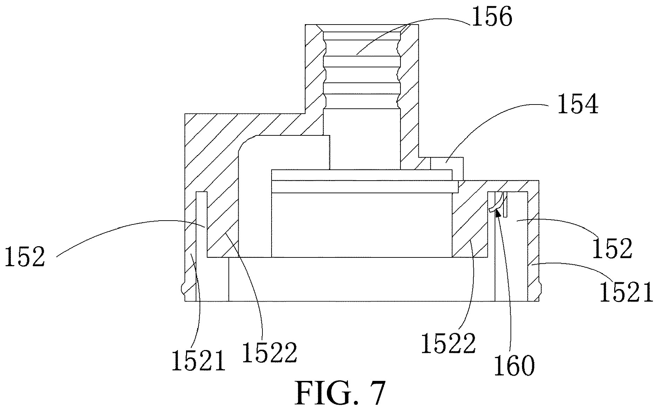

As shown in FIGS. 3, 6, and 7, FIG. 6 is a schematic structural view of an embodiment of the sealing member shown in FIG. 2, and FIG. 7 is a cross-sectional view of the sealing member shown in FIG. 6.

The baffle plate 160 may be arranged in the first intercepting groove 142. The baffle plate 160 may close the first intercepting groove 142 in a natural state in which no air is inhaled from the atomization cavity 121 through the air outlet channel 123, and disconnect communication between the air inlet channel 122 and the atomization cavity 121; and the baffle plate 160 opens the first intercepting groove 142 when an inhaler inhales air from the atomization cavity 121 through the air outlet channel 123, to communicate the air inlet channel 122 with the atomization cavity 121.

In some embodiments, the baffle plate 160 may be mounted at a joint between the atomization cavity 121 and the air inlet channel 122, and an opening at the joint between the atomization cavity 121 and the air inlet channel 122 may be sealed by the baffle plate 160, thereby reducing a risk of the liquid in the atomization cavity 121 leaking into the air inlet channel 122.

In some embodiments, the baffle plate 160 may be mounted on the side of the sealing member 150 close to the base 140. When the base 140 is connected to the sealing member 150, the baffle plate 160 may be partially inserted into the first intercepting groove 142. In addition, when the baffle plate 160 is in a natural state, the baffle plate 160 may abut against or substantially abut against the bottom wall and the side wall of the first intercepting groove 142, and cut off the first intercepting groove 142.

When the inhaler inhales air from the atomization cavity 121 through the air outlet channel 123, the air pressure in the atomization cavity 121 decreases. The baffle plate 160 is bent and deformed under the action of a pressure difference between the air inlet channel 122 and the atomization cavity 121, to cause the first intercepting groove 142 to be opened, in this way, the air inlet channel 122 is in communication with the atomization cavity 121. In this case, the external air may enter the atomization cavity 121 along the air inlet channel 122, and then be sucked out along the air outlet channel 123. Therefore, an airflow may be generated in the atomization cavity 121, and aerosol of the atomized liquid in the atomization cavity 121 is sucked out from the air outlet channel 123 along with the airflow.

When the inhaler stops inhaling air from the atomization cavity 121 through the air outlet channel 123, the baffle plate 160 may gradually return to a natural state, to close the first intercepting groove 142. Therefore, liquid in the atomization cavity 121 (such as condensate of the atomized liquid) may be prevented from leaking from the air inlet channel 122.

In some embodiments, the baffle plate 160 may be integrally formed with the sealing member 150. Alternatively, each of the baffle plate 160 and the sealing member 150 may be an independent component, and the baffle plate 160 and the sealing member 150 may be assembled after they are respectively formed.

In some embodiments, the baffle plate 160 may further be mounted on the base 140. Specifically, the baffle plate 160 may be mounted on the bottom wall of the first intercepting groove 142. When the sealing member 150 is mounted on the base 140 and is engaged with and fixed to the base 140, the baffle plate 160 may abut against or substantially abut against the bottom wall and the side wall of the sealing member 150. In this way, the baffle plate 160 may close the air inlet channel 122 formed by the first intercepting groove 142 and the sealing member 150 in a natural state, and disconnect communication between the air inlet channel 122 and the atomization cavity 121 at a location of the first intercepting groove 142.

Reference is further made to FIGS. 6-7.

An engagement groove 152 is provided on the sealing member 150, and the engagement groove 152 is sleeved on the side wall of the base 140, in this way, the sealing member 150 may be engaged with the side wall of the base 140 through the engagement groove 152, to fix the base 140 to the sealing member 150.

The engagement groove 152 may be provided on the side wall of the sealing member 150, and the side wall of the base 140 may be inserted into the engagement groove 152. Specifically, the side wall 1521 on one side of the engagement groove 152 may be arranged between the air guiding groove 141 and the side wall of the mounting cavity 112, and engage with the air guiding groove 141 to form the air inlet channel 122. The side wall 1522 on the other side of the engagement groove 152 is arranged between the atomization core 130 and the side wall of the base 140.

In some embodiments, the sealing member 150 may be formed by a flexible material such as silica gel or rubber. When the mounting base 120 is mounted in the mounting cavity 112 of the atomization sleeve 110, the sealing member 150 may fill a gap between the base 140 and the inner wall of the atomization sleeve 110, and seal a location of the mounting cavity 112 of the atomization sleeve 110.

A liquid feeding channel 154 and an air outlet pipeline 156 are further provided on the side of the sealing member 150 close to the liquid storage cavity 111 of the atomization sleeve 110. The liquid feeding channel 154 may be docked with the liquid storage cavity 111. When the atomization core 130 is arranged in the atomization cavity 121, the atomization core 130 may be covered on an opening of the liquid feeding channel 154 located in the atomization cavity 121, in this way, a liquid absorbing surface of the atomization core 130 is at least partially exposed to the liquid storage cavity 111 through the liquid feeding channel 154, and the liquid absorbing surface of the atomization core 130 is in contact with the atomized liquid stored in the liquid storage cavity 111. Therefore, the atomized liquid in the liquid storage cavity 111 may be heated and atomized by the atomization core 130 to form aerosol of the atomized liquid in the atomization cavity 121.

One end of the air outlet pipeline 156 may be docked with an inhalation pipeline 113 in the atomization sleeve 110, and the other end of the air outlet pipeline 156 may be in communication with the air outlet channel 123. That is, aerosol formed into the atomized liquid in the atomization cavity 121 may be sucked out from the inhalation pipeline 113 along the air outlet channel 123 and an air outlet pipeline 156.

As shown in FIGS. 3 and 5, an electrode hole 144 may be further provided on the side of the bottom wall of the base 140. The electrode hole 144 is configured to arrange a conductive member 170, and one end of the conductive member 170 may be inserted into the atomization cavity 121 through the electrode hole 144 to be electrically connected to the atomization core 130 therein. The other end of the conductive member 170 may be exposed from the side of the bottom wall of the base 140 through the electrode hole 144. The other end of the conductive member 170 may be electrically connected to an external power supply, to supply power to the atomization core 130, in this way, the atomization core 130 heats and atomizes the atomized liquid to form aerosol of the atomized liquid in the atomization cavity 121.

Further, a bottom air inlet notch 145 is further provided on the bottom wall of the base 140, the bottom air inlet notch 145 may be in communication with the external air. An air guiding groove 141 on the side wall of the base 140 may be extended to be in communication with the bottom air inlet notch 145.

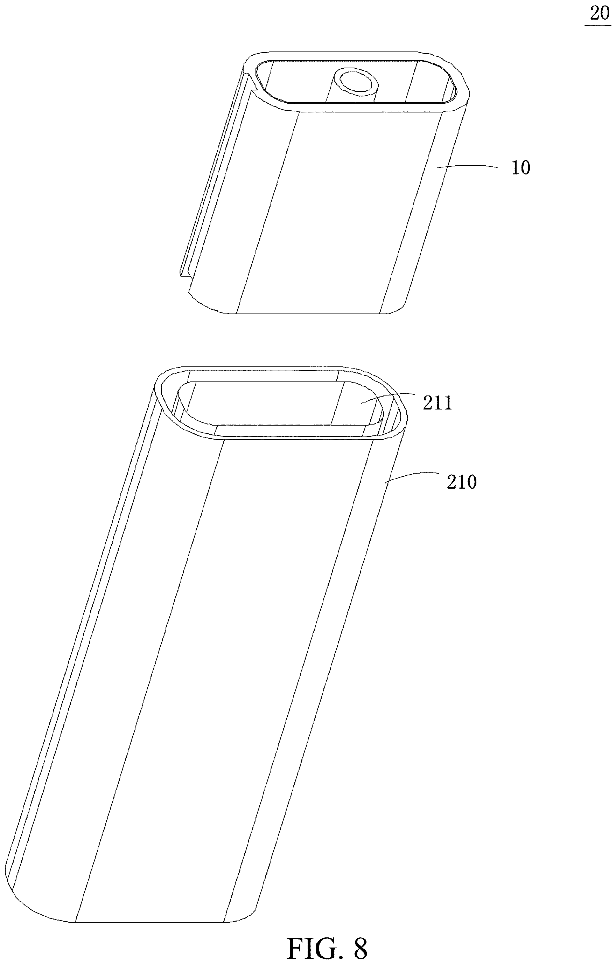

The present disclosure further provides an electronic atomization device. As shown in FIG. 8, FIG. 8 is a schematic structural view of an embodiment of an electronic atomization device according to the present disclosure.

The electronic atomization device 20 includes an atomizer 10 and a body assembly 210. The atomizer 10 may be configured to store the atomized liquid and atomize the atomized liquid to form an aerosol that may be inhaled by the inhaler. The atomizer 10 may be mounted on the body assembly 210, and a power supply assembly is arranged in the body assembly 210. When the atomizer 10 is mounted on the body assembly 210, positive and negative poles of the power supply assembly in the body assembly 210 may be respectively electrically connected to two conductive members 170 in the atomizer 10, so as to form a power supply circuit to supply power to a linear atomization core 130.

A mounting groove 211 is provided on the body assembly 210. The side of the atomizer 10 close to the bottom wall of the base 140 may be inserted into the mounting groove 211 to be fixed to the body assembly 210.

In summary, a person skilled in the art may easily understand that beneficial effects of the present disclosure are as follows: the present disclosure provides an electronic atomization device and an atomizer thereof. By providing at least one fine slot in an air inlet channel, condensed atomized liquid in the air inlet channel may enter the at least one fine slot for buffering, thereby preventing the condensed atomized liquid from leaking out from an air inlet of the air inlet channel. Further, by providing the air inlet channel on either end of long sides of the cross section of an atomization cavity, when an atomization core is mounted in the atomization cavity, a path of passing an atomization surface of the atomization core performed by an airflow entering the atomization cavity from the air inlet channel may be lengthened, thereby increasing a heating time of the airflow. In this way, condensate of atomized liquid and splashed atomized liquid generated in an atomizer may be fully absorbed or heated and atomized by the atomization core, reducing occurrence of a phenomenon of inhalation leakage and condensate accumulation.

The foregoing descriptions are merely embodiments of the present disclosure, and the protection scope of the present disclosure is not limited thereto. All equivalent structure or process changes made according to the content of this specification and accompanying drawings in the present disclosure or by directly or indirectly applying the present disclosure in other related technical fields shall fall within the protection scope of the present disclosure.

## Claims

1. An atomizer, **characterized by** comprising:
an atomization sleeve, wherein a liquid storage cavity and a mounting cavity are provided on the atomization sleeve;
a mounting base, wherein the mounting base is arranged in the mounting cavity, and an atomization cavity, an air inlet channel in communication with the atomization cavity, and an air outlet channel in communication with the atomization cavity are provided on the mounting base; and
an atomization core, wherein the atomization core is mounted in the atomization cavity;
wherein at least one fine slot is provided on the inner wall of the air inlet channel, and the at least one fine slot is configured to buffer liquid entering the air inlet channel.

2. The atomizer according to claim 1, wherein
the mounting base comprises a base and a sealing member, the sealing member is arranged on the side of the base close to the liquid storage cavity, and is engaged with the base, and the sealing member and the base are enclosed to form the atomization cavity.

3. The atomizer according to claim 2, wherein
an air guiding groove is provided on the outer surface of the side wall of the base, the air inlet channel is formed by the air guiding groove and the side wall of the mounting cavity cooperatively, the end of the air inlet channel close to the liquid storage cavity is in communication with the atomization cavity, and the end of the air inlet channel away from the liquid storage cavity is in communication with external air; and
the at least one fine slot is provided in the air guiding groove.

4. The atomizer according to claim 3, wherein
the air guiding groove is extended along the side of the mounting base close to the liquid storage cavity to the side away from the liquid storage cavity, and the at least one fine slot is provided on the bottom wall of the air guiding groove and the width of the at least one fine slot ranges from 0.2 mm to 1.2 mm.

5. The atomizer according to claim 3, wherein
the air guiding groove is extended along the side of the mounting base close to the liquid storage cavity to the side away from the liquid storage cavity, and the at least one fine slot is provided on the bottom wall of the air guiding groove and the width of the at least one fine slot ranges from 0.3 mm to 0.7 mm.

6. The atomizer according to claim 4, wherein
a first intercepting groove is provided on the side of the air guiding groove close to the liquid storage cavity, and the at least one fine slot is in communication with the atomization cavity through the first intercepting groove.

7. The atomizer according to claim 6, wherein
a second intercepting groove is provided on the side of the air guiding groove away from the liquid storage cavity, and the at least one fine slot is in communication with the external air through the second intercepting groove.

8. The atomizer according to claim 4, wherein
at least one third intercepting groove is provided in the middle portion of the air guiding groove, and the third intercepting groove cuts off the at least one fine slot.

9. The atomizer according to claim 7, wherein
the first intercepting groove is a first notch on the edge of the side wall of the base close to the liquid storage cavity, and the second intercepting groove is a second notch on the edge of the bottom wall of the mounting base.

10. The atomizer according to claim 2, wherein
a baffle plate is arranged between the air inlet channel and the atomization cavity;
the baffle plate is configured to close the air inlet channel in response to no air being inhaled from the atomization cavity through the air outlet channel, and disconnect the communication between the air inlet channel and the atomization cavity; and
the baffle plate is configured to open the air inlet channel in response to air being inhaled from the atomization cavity through the air outlet channel, to communicate the air inlet channel with the atomization cavity.

11. The atomizer according to claim 10, wherein
an engagement groove is provided on the side wall of the sealing member, and the engagement groove is sleeved on the side wall of the base; and the side wall on one side of the engagement groove is arranged between the air inlet channel and the side wall of the mounting cavity, and the side wall on the other side of the engagement groove is arranged between the atomization core and the side wall of the base.

12. The atomizer according to claim 11, wherein
the baffle plate is fixed to the bottom wall of the engagement groove of the sealing member, and the baffle plate is integrally formed with the sealing member.

13. The atomizer according to any one of claims 1 to 12, wherein
the cross section of the atomization cavity has two opposite long sides and two opposite short sides, and the air inlet channel is located on either end of the long sides.

14. An electronic atomization device, **characterized by** comprising:
an atomizer, wherein the atomizer is configured to store liquid to be atomized and atomize the liquid to form an aerosol for inhalation by an inhaler, and the atomizer is the atomizer according to any one of claims 1 to 13; and
a body assembly, wherein the body assembly is configured to supply power to the atomizer.
